# EUROPEAN PATENT APPLICATION

(11) **EP 1 304 082 A2**
(43) Date of publication of application: **23.04.2003**
(21) Application number: 01954532.6
(22) Date of filing: 17.07.2001
(51) Int. Cl.: A61B 17/58

(54) **DEVICE AND METHOD FOR BONE FIXATION, COMPRESSION AND DISTRACTION**

(30) Priority: 17.07.2000 MX 0007016
(71) Applicant: Flores Davila, Jorge P., Toluca, México 55120 (MX)
(72) Inventor: Flores Davila, Jorge P., Toluca, México 55120 (MX)
(74) Representative: Stenger, Watzke & Ring Patentanwälte
(86) International application number: MX0100050
(87) International publication number: WO02007620

(57) **Abstract**

A double plate for bone fixation, compression and separation is described. The double plate comprising: a fixation plate 10 having (a) holes 13 in which cortical screws are placed to fix the plate 10 to the first bone segment, (b) at least one groove 12 that allows the movement of the cortical screws that are placed on the sliding plate 20 which is fixed on the second bone segment; a sliding plate 20 having (a) holes 23 where cortical screws are placed to fix the plate 20 to the second bone segment, (b) a groove 22 where cortical nails are placed to fix the fixation plate 10 to the fist bone segment; and a mechanism to generate and transmit movement 18 that will allow the sliding plate 20 to move lengthwise in relation to the fixation plate 10. The direction in which the movement is set will result in compression of separation of the bone segments.

Using the invented device and procedure, a stable, dynamic, and permanent osteosynthesis can be achieved, in other words, a controlled interfragmentary compression on the force that is exerted on the fragments and importantly, that this is applied during the entire consolidation process, reviving in an effective manner the ideal requirements to obtain an adequate consolidation.

## Description

### Background Information

### CONCEPTS

### Bone Compression and Separation.

According to scientifically based studies done on the effects caused on the bone structure when compression and separation techniques are done; the use device and procedure of the present invention gives one remarkable results in the above mentioned procedures in relation to bone consolidation through compression and bone elongation on the bone segments that require extension for a better function.

### FRACTURES

A fracture is the discontinuity of the bone structure due to unknown etiologies or causes.

Fractures generally are caused by traumatic, pathologic, surgical or jatrogenic factors whose mechanisms or alterations produced on the bone lead to loss of bone continuity as before commented.

### Fracture Classification:

Basically depending en the mechanism of this type of lesion, various fracture forms or fragments which can be classified according to different parameters:
a) In relation to the state of the surrounding soft tissue:
   - Closed or not exposed.
   - Opened, or exposed.
b) According to the type of force causing the fracture:
   - Direct.
   - Indirect.

The indirect fractures can be:
- Due to a twisting force- Spiral fracture-.
- Due to an angular force- Transverse fracture-.
- Due to a combination of an angular force with central compression producing fractures of variables sizes and shapes but that generally are triangular called "butterfly wings".

The direct fractures can be:
- Due to a blow which produces transversal fractures and some degree of skin damage.
- Due to crushing which usually results in comminuted bone fragments.

The above mentioned fractures usually occur in long bones. In the short or spongy bones like the tarsal or carpal bones, the comminuted fractures are caused by crushing forces.

There also exists fractures caused by avulsion, for example, in the knee or olecranon, when resistance occurs to the muscular action.

### PATHOLOGIC ANATOMY

The fractures produce loss of histiocyte continuity in the bone tissue.
The bones are functional elements having a particular elasticity, resistance, hardness; etc. The bones are formed by various elements which determine their physiological properties which when altered cause different pathologies which in turn frequently cause loss of continuity of the bone structure, eventually producing fractures.

Currently, due to histological and imagery findings, the structural, biochemical and metabolic composition of the bone has been determined, but most importantly are the findings on its physiological characteristics, pathology and treatment.

### CLINICAL ASPECTS OF FRACTURES

A fracture is usually followed by pain, functional incapacity, deformity of the region, changes in the axis in the case of long bones and several manifestations in the adjacent soft tissue.

### DIAGNOSIS

The diagnosis of a fracture is basically assessed when the above clinical signs appear at the moment of inspection palpation, and the execution of certain diagnostic maneuvers which will put in evidence any crepitus, abnormal movements and alteration of the axis of the damaged segment.

The current imagery studies are of great importance to understanding the details of the lesions in the fractures which will lead to the best solution in the treatment.

### FRACTURE TREATMENT

There is an important difference between the treatment of close fractures and open fractures.

With the final results in mind, the objective which the orthopedist pursues when locking for a solution to a fracture is to achieve a consolidation of the bone fragments arranging these in their anatomical and or functional position through an immobilization technique according to accepted standard procedures.

In exposed fractures, the fist treatment step is to protect the segment from the damaging components of the lesion, especially when vital elements of the member or affected region are at risk; this is important to keep in mind as well as the fact that these type of fractures tend to develop various complications, infection being one frequent and which should be avoided.

According to the above mentioned in relation to exposed fractures, it's also fundamental to take in consideration the following:

Preservation of the vital elements of the affected member, reduction of the danger of shock, cleansing of the wound through debridation of the affected tissues, elimination of necrotic tissue, and immobilization of the segment, preferably through external immobilization once the vitality, irrigation, function and protection of he peripheral nerves in the articulations and the rest of the member have been assured.

### TREATMENT OF CLOSED FRACTURES

Closed fractures are basically treated through: reduction, external and or internal fixation, and rehabilitation.
In accordance with the previously described, the device and procedure of the present invention are guided to offer a new proposal and its basis in the application of a novel solution to the treatment of dosed and exposed fractures based en the good criteria of the treating doctor.

Following is a brief description of the external and internal fixation of the prior art:

In selecting a fixation technique we have:
1. Traditional plate: Perforated Egger, Venable or Barns plates, which are not used very much.
2. Compression plates: Dynamic compression Plates (DCP) comprising oval holes.
3. Protection plates: Spiral and comminuted fractures can be fixed with screws, but it's important to compliment this and give protection using a plate.
4. Multiple plates: Some fractures require fixation with multiple plates, their use is not widespread, the advantage using this plates is that they offer the most resistance.

All these plates function basically immobilize the union between the fractured fragments to allow cellular bone regeneration. This regeneration permits recuperation of the cohesion of the affected segments.

The force with which the screws are applied In the plates (2, 000-4,000 N) produces between the fractured parts, in proportion to the Poisson effect, a force of 600-1,000 N. This compression, which in large produces the solidification of the fracture, is affected in a negative manner by the "lysis" of the very same fracture.

### MARROW SPLINTS

Choosing from various osteosynthesis procedures for the application of an internal splint depends en the nature of the fracture, of the material available and on the criteria of the orthopedist. Also exists opened and closed centromedullary nails.

### FIXATION ELEMENTS FOR MARROW SPLINTS

Rigid nails, flexible nails, Kirschner wires, nails, plates and screws. Other fixation techniques, in some cases, is with wire cerclages.

From the aforementioned procedures, the compression plate and the devices for external fixation are preferred by traumatologists and orthopedists.

### BIOLOGICAL ASPECTS THAT HAVE AN IMPORTANT INFLUENCE IN BONE COSOLIDATION:

The following factors determine the quality as well as the velocity with which a fracture heals; according to this, the fracture can heal quickly and in optimum condition or it can have setbacks in consolidating or not consolidate at all which is known as "Pseudo Arthrosis":
- Antinflamatory drugs due to their inhibition of prostaglandins.
- Compression or separation of the fractured segments:
   it's known that the interfragmentary compression during the consolidation process produces electronegative charges, which accelerate consolidation to such a magnitude that according to studies done on this compression process the consolidation observed in human beings was obtained in a significant decreased time.

Other factors that influence in consolidation are:
- The action of sensitive nerves.
- The degree of hemorrhage and the quality of the circulation of the blood.
- The presence of phagocytes, mast cells and mononuclear cells.
- The necrosis in the tissue surrounding the fracture.
- The interposition of tissue between the fracture fragments.
- The activity of the osteoblasts.
- The state in which the periostium and the bone marrow cavity are in.
- The state of quality of the organic tissue and its response with histiocyte proliferation.
- The alignment of the fractured segments.
- The degree of stability of the fracture, which is one of the main issues in the process of consolidation.
- The interfragmentary compression, a basic aspect: ideally a continuous intefragmantary compression during the entire process of consolidation.

It's also been proven that other factors like; ischemia, hypoxia, and the increase of pCO₂ intervene in the process of bone consolidation.

The degree of bone separation is so important that it has been pointed out that if there exists a space of 0.5 cm between two fractured bone segments, it would take one year of histiocyte continuity to achieve consolidation.

Other factors that contribute to bona consolidation are the activation of enzymes such as thoe related to alkaline phosphate, glycosamine glucans, hyaluronic acid, hexosamine, and hydrexyproline.

### Description of the Invention.

The task of this invention consists in providing a device and procedure for the fixation and compression, or fixation and separation of the bone segments, which will allow sliding between the fractured segments or the separation of these segments.

The aforementioned objective, is achieved through the use of the plate of the invention. The fundamental characteristic of the plate consists in providing a plate that while it's fixed to the fractured segments, it will also permit compression or separation of the same segments. This compression or separation can be controlled, or better said, can be freely manipulated to establish a condition of controlled compression or controlled separation between the fractured fragments.

Even though the invention is described using a bone fracture of two segments, it would be obvious for a skilled in the art, that the proposed procedure and device can be applied in fractures with multiple fragments.

The device and procedure of the present invention is focused towards its use on fractures where compression is used on the bone segments, however, the device can also be used in procedures where separation of the bone segments is necessary.

In relation to the present invention, bone compression is referred to the application of a central or axial force on each bone segment so that the bone fragments joint in the best manner, under optimum consolidation conditions, and in the shortest time possible.

Bone separation on the other hand, refers to the application of a central or axial separating force en the bone segments which pulls apart one bone fragment from the other.

While bone compression has its use basically in bone fractures, bone separation finds its use in procedures that try to achieve extension or growth of the bone. This device, using the same principle, achieves both objectives depending on the direction the force is applied.

### DESCRIPTION OF THE FIGURES

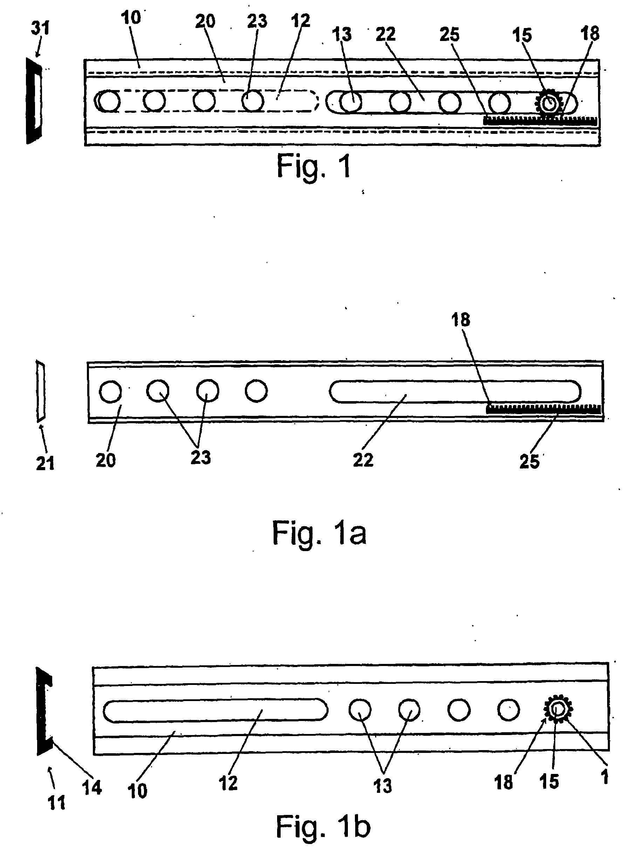

Figure 1 illustrates a double plate used. according to the first procedure embodiment proposed in the present invention.
Figure 1.A illustrates the first component of the plate from Fig. 1.
Figure 1.B shows the second component of the same plate from Fig. 1.
Figure 2 illustrates the use of the plate in accordance with the second embodiment of the present invention.

### DETAILED DESCRIPTION OF THE INVENTION.

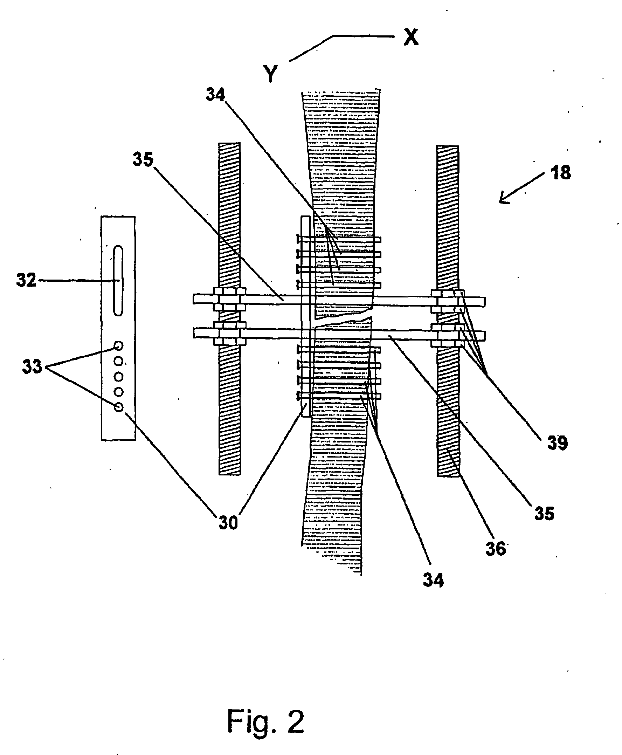

According to the present invention, the fractured bone segments are provided with a support that consists of a plate with a groove or slot. The function of this plate is to oppose the shearing forces that exist in the XY plane where the fractures occur, as is shown in Fig. 2. The fundamental feature of the plate in the present invention lies in the incorporation of a groove which in combination with means that produces and transmits movement, allows for bone compression and separation.

First and second embodiments of the invention are hereinafter disclosed, with reference to the compression of bone segments.

Referring to Fig. 1, 1.A and 1.B the assembled double plate is shown. The double plate comprises a fixation plate 10 and a sliding plate 20. Number 11 and 21 are cross sectional views of each plate where one can see that the plate 10 forms a ridge 14 where the sliding plate 20 can fit inside the fixation plate 10 so that the sliding plate can only move longitudinally according to the space formed by the ridge 14 which functions as a guide for the sliding plate 20.

The advantage of this invention is that these plates can provide controlled interfragmentary compression throughout the entire bone consolidation process.

According to the first embodiment, cortex or cortical screws are placed in the holes 13 of the fixation plate 10 segment. The holes 23 on the sliding plate 20 have a similar function when used en the second fractured bone segment.

The fixation plate 10 has a groove 12. The screws in the holes 23 on the sliding plate 20 must cross the fixation plate 20 without blocking the sliding plate. The groove 12 allows the sliding plate 20 to move even though the screws have been placed in the holes 23.

The sliding plate 20 has a groove 22 designed so that the cortex or cortical screws can be placed in the holes 13 on the fixation plate 10, these screws must be at the same level of the fixation plate 10 to allow the sliding plate 20 to move.

The compression and separation of the bone segments is obtained through means that produces and transmits movement 18, according to the first embodiment of the invention such means 18 comprises the circular gear 15 and the straight gear 25. The circular gear 15 is attached to the plate 10 and works in combination with the straight gear 25 en the plate 20, the circular gear 15 transmits movement to the plate 20 which allows this plate to move and thereby attains interfragmentary compression. This action is due to the plate 10, which is a fixed plate with the gear system before mentioned, and the plate 20, with its structure, which allows a fractured bone segment to slide, once its reduced, producing interfragmentary compression.

The gear 15 is placed on the fixation plate 10, while the straight gear 25 is located en the sliding plate 20. When the fixation plate 10 and the sliding plate 20 are assembled, the gears 15 and 25 are put in contact in such a way that when the gear 15 is turned, a longitudinal movement of the plate 20 is produced. The direction in which the gear 15 is turned determines the direction the sliding plate 20 will take which in turn will causes compression or separation of the fractured segments.

The straight gear 25 has the sufficient length to allow the plate 20 to move, a movement of 2 cm is sufficient enough to obtain compression of the bone segments. In Fig. 1, one can see that the gear 15 is found inside the groove 22 of the plate 20, however, an independent groove can be provided solely for the movement of the gears.

To prevent the natural separation of the fractured fragments, the gears 15 and 25 have teeth with an specific angle, this angle allows the plate 10 and 20 to move in only one direction. Therefore, the gears should be fashioned to tolerate the force about of 200 KPa applied to the site of the interfragmentary compression.

Due to the double width of the double plate, the fractured segments are provided with an extraordinary reinforcement against the shattering forces acting en the fractured plane, this reinforcement has a direct influence in the early rehabilitation of the patient because the plate restores to the bone the strength lost due to the fracture. So, even though the patient may have a fractured femur that has not completely consolidated, it will be possible for the patient to walk as result of thanks the use of this plate and to the interfragmentary compression.

The gear 15 on the double plate discussed in the first proposed application procedure, sticks out approximately 2 cm from the fractured member, in this manner, a discrete and functional orthopedic device is provided.

The second embodiment of the invention is illustrated in Fig. 2, and requires only one plate that works with the same principle described above, except that in this system there are no gears or a central internal compression, as was earlier mentioned. Compression in this instance is done using external compressors and transosseous nails, for example, external compressors and Steinmann nails. As mentioned, this embodiment uses only one plate.

Referring now to Fig. 2, the device used in the second embodiment comprises a plate 30 that has holes 33 for cortex or cortical nails, the nails 34 fix the bone segments to the plate 30 to give reinforcement against the perpendicular shearing forces acting en the fracture plate. The plate 30, as in the case of the plates used in accordance with the first embodiment of the invention shown if Fig. 1, also has a groove 32, which permits movement of the fractured fragments and the fixing nails. In the second embodiment, Fig. 2 shows how compression and separation of the fractured segments is obtained through the use of 2 transosseous nails, these nails pass through each fractured segments (proximal and distal) and soft tissues and are externally connected with the means to produces and transmits movement 18 that will cause compression and/or separation of these segments. So that this movement can occur, one of the mentioned nails must be fixed, this Is done using a nail 35 in one of the holes 33, this nail should preferably be placed in the hole closest to the groove 32, while the second nail is placed inside the groove 32. The number of cortex nails used depends on the size of the fractured. bone, usually 3-4 are used en each fractured segment.
The means 18 can be formed by any means known in the art used to generate movement in one direction, for example one can use a hydraulic or pneumatic piston or any other mechanical means. In accordance with the embodiment of Fig. 2, the means 18 comprises two threaded bars 36 placed at the end of each nail 35. The threaded bar comprises of bolts 39 that screw onto the threaded bar 36, each bar also has a groove that forms an space in part of the length of the bar where the ends of the nails 35 are inserted. As can be appreciated in Fig.2, each end of the nail 35 is surrounded by a pair of bolts 39. Toe produce and transmit movement, the first bolt is first turned and then the second bolt is turned, as each bolt is turned, a longitudinal movement is produced along the bar 36, this movement is transmitted to the nails which in turn transmit the movement to the bone segments. Depending on the direction that is given, this movement is translated into a compressing force or a separating force en the bone segments.

This movement is only possible through the grooves 32 found on the plates 30, the grooves 32 permit at least one trans- cortical nail to move through the grooves 32 which serve as guides for these same nails 35. Fig. 2 shows the plate with a groove 32 placed on the upper end of the plate, however, as it can be obvious to those skilled in the art, the groove can be placed anywhere along the plate 30. The plate can have many grooves and can be used to unite more than two bone segments, special designs of this plate depend on the specific application in mind, however, said options in the design of the holes and grooves are considered included within the scope of the invention. Furthermore, the plate in the same figure is shown as being flat or relatively flat, however, it can be given a slightly curved surface that follows the curvature of the bone surface which will yield similar results.

### ADVANTAGES OF THE PROCEDURE

According to the processes of bone reparation of the prior art, some of the most important aspects in obtaining good bone consolidation are:
- Suitable treatment election.
- Stable osteosynthesis.
- Will controlled Interfragmentary compression until consolidation is obtained.
- Preservation of the vital functions of the tissue involved.
- Early rehabilitation.
- Correct surgical access to the site and adequate handling of the tissues.
- Use of a guide to avoid misalignment of the bone axis.

The above mentioned parameters are vital in order to achieve a rapid and maximum quality of consolidation, as well as a quick recuperation of a anatomically functioning segment.

The proposed procedure fundamentally tries to solve, in the best possible manner said parameters.

### JUSTIFICATION

The present invented device and procedure pretends to:
1. Resolve a innumerable cases of bone fracture which daily occurs; throughout the world and which will keep on increasing in number due to the development of technology and mechanization.
2. Propose a new system whereby the mechanics of its elements achieves a fixation that is:
   - Stable.
   - Easy to apply.
   - Allows a rapid rehabilitation, and the possibility of applying a process of compression during the entire period of consolidation, checked periodically by clinical and radiographic studies.

This procedure is simple, exerting compression using only an external compression device, or using axial or central compression applied on the gear mentioned above.

| CASE STUDY: | |
|---|---|
| *Patient name:* | PCT. |
| *Age:* | 37 years oid. |
| *Diagnosis:* | Multifragmented fracture in the middle, inferior one third of the left femur. |
| *Time spent since the occurrence of the fracture* | 6 months. |
| *Details of the operation previously performed:* | central medullar femoral universal nail with 2 proximal screws and 2 distal screws operation p-1Xe weeks after the fracture occurred. |
| *Patient evolution:* | breakage of the nail at the height of the proximal screw in the distal segment provoking bone separation up to 1 cm between the fractured segments. |
| *Corrective surgery:* | Application of the bone fixation compression plate of the present invention 6 months after the fracture originally occurred. |
| *Corrective operating technique performed* | Application of the fixation-separating plate for compression with external screws and Steinmann nails according to the second embodiment of the invention, as is illustrated in Fig.2. |
| *First steps of walking* | are taken 16 days after the corrective surgery was performed. |
| *Withdrawal of the external compressors* | is done 5 weeks after the surgery. The patient continues with good evolution and is able to walk satisfactorily sustaining his body weight on the operated member using a stroller. During his rehabilitation, which started 16 days after the corrective surgery, the patient never complained of pain in the fracture site and only mentioned minimal pain in the soft tissues. |
| *Stability* | sustains body weight using a stroller. |

### EVOLUTION

- Stable fixation.
- Good evolution of the soft tissues.
- Slight to moderate edema.
- Using a stroller, the patient can walk 16 days after the surgery.
- Stable gait and without pain.
- Control X-ray shows osteogenic bone consolidation or per primam.
- Increase of bone density on the site of the fracture.

### PROCEDURE

The application is simple:
1. Screws are placed in the fixation plate 10.
2. Screws are placed in the sliding part of the plate
3. Compression is exerted using the external compressors (second embodiment) or through the axial or central compression produced by the gears (first embodiment)

### ADVANTAGES

This procedure achieves primary union or per primam union, which due to the permanent interosseos compression, obtains a dilation of the Haversian ducts on both sides of the cut bone; internal canalization directed toward the fracture line to cross this with osteoclast cones to obtain osteoblast deposits and formation of a bone callus.

The compression-sliding plate or double fixation sliding plate for the manually controlled compression system:
1. Avoids lack of union or lags in the bone consolidation.
2. Achieves an osteogenic consolidation that produces a bone callus of optimum quality.
3. Complete stability and functional recuperation that avoids prolonged incapacity, degeneration of the tissues due to prolonged rest, and pathologies due to the fracture.
4. Lowering of costs due to the rapid recovery of the patient.

The characteristics of the invented device and procedure also offer:
- -A system of manually controlled compression that is continuous until the complete consolidation is achieved.
- Firm fixation.
- Early recuperation with a decrease in sequelea caused by a prolonged immobilization
- Using the same device, but exerting opposing forces instead of compressing forces, produces separation of the bone segments which allows for bone extension in the same affected member using a procedure whose mechanical function provides the best comfort, security and stability.

### INDICATIONS OF THE PROCEDURE

The system of compression can be applied to any fracture, that due to the shape of the bone segments in the fracture, can fit the plates mentioned in the two embodiments of the invention. These same embodiments can be applied to any use that is given to the compression plates with oval holes of the prior art.

### SECURITY

The compression force that is applied to a fracture should not cause additional fissures. Also should be considered the *Lysis* of the fracture as well as the compression lost by the Poisson Effect should be considered.

To obtain the advantages of the present invention, a deformation by compression of approximately 0.43 mm is required in patients 20 years old or less and of 0.21 mm in patients 70 years old. The relationship between deformation and age is approximately lineal.

## Claims

1. Double plate for bone fixation, compression and separation comprising:
- a fixation plate 10 having (a) holes 13 in which are placed cortical nails to fix the plate 10 to the first fractured bone segment, (b) at least one groove 12 which allows the cortical nails which are used to fix the sliding plate 20 to the second fractured segment, to move;
- a sliding plate 20 having (a) holes 23 where cortical screws are placed to fix this same plate 20 onto the second fractured segment, (b) a groove 22 that allow the placement of the cortical screws that fix the fixation plate 10 onto the first fractured segment; and
- a mechanism for the production and transmission of movement 18 that allows the sliding plate 20 to move lengthwise in relation to the fixation plate 10. The direction in which the movement is set results In compression or separation of the bone segments.

2. Double plate for bone fixation, compression and separation according to daim 1, wherein the mechanism that produces movement and transmits it is formed by a gear 15 found on the fixation plate 10, and a straight gear 25 found on the sliding plate 20.

3. Double plate for bone fixation, compression and separation according to claim 1, **characterized by** the fixation plate that forms ridges 14 that serve as guides for the movement of the sliding plate 20.

4. A device for bone compression and separation formed by:
- a plate 30 with holes 33 and at least one groove 32, to place cortical screws 34 that fix the plate to the bone segments;
- one transcortical nail that is placed in the hole 33 of the plate and remains in a fixed position.
- at least a second transcortical nail 35 that is mobile and passes through the plate through the groove 32; and
- a mechanism 18 that produces movement placed on the ends of the transcortical nails which will produce compression or separation of the segments.

5. A device for bone compression and separation according to claim 4, wherein the mechanism 18 comprising:
- a pair of threaded bars 36 placed on each end of the nails 35, each bar 36 also has a groove that has a space in part of the length of the bar 36, in this space the ends of the nails 35 are introduced; and
- bolts 39 placed on the threaded bar 36, each end of the nail 35 is surrounded by a pair of bolts 39. To produce and transmit movement, first one bolt is turned and next a second bolt is turned, turning each bolt causes a longitudinal movement along the length of the bar 36, this movement is transmitted to the nails whichin turn is transmitted to the bone segments.
